(19) Europäisches Patentamt
European Patent Office
Office européen des brevets

(11) Veröffentlichungsnummer: **0 033 716**
**B1**

(12) # EUROPÄISCHE PATENTSCHRIFT

(45) Veröffentlichungstag der Patentschrift: **25.05.83**

(21) Anmeldenummer: **81810019.0**

(22) Anmeldetag: **26.01.81**

(51) Int. Cl.³: **C 07 D 239/74,** C 07 D 239/91, C 07 D 239/93, C 07 D 239/94, C 07 D 403/02, C 07 D 401/12, C 09 B 62/20, B 41 M 5/16, B 41 M 5/18

(54) **Chromogene Chinazolinverbindungen, Verfahren zu ihrer Herstellung und ihre Verwendung als Farbbildner in druckempfindlichen oder wärmeempfindlichen Aufzeichnungsmaterialien.**

(30) Priorität: **31.01.80 CH 780/80**
**15.07.80 CH 5411/80**

(43) Veröffentlichungstag der Anmeldung: **12.08.81 Patentblatt 81/32**

(45) Bekanntmachung des Hinweises auf die Patenterteilung: **25.05.83 Patentblatt 83/21**

(84) Benannte Vertragsstaaten: **AT BE CH DE FR GB IT LI**

(56) Entgegenhaltungen: **DE-A-1 795 748**
**DE-B-1 172 266**

(73) Patentinhaber: **CIBA-GEIGY AG, Patentabteilung Postfach, CH-4002 Basel (CH)**

(72) Erfinder: **Fletcher, Ian John, Dr., Bürgenstal 27, CH-4465 Magden (CH)**

EP 0 033 716 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Chromogene Chinazolinverbindungen, Verfahren zur ihrer Herstellung und ihre Verwendung als Farbbildner in druckempfindlichen oder wärmeempfindlichen Aufzeichnungsmaterialien

Die vorliegende Erfindung betrifft chromogene Chinazolinverbindungen, Verfahren zu ihrer Herstellung und ihre Verwendung als Farbbildner in druckempfindlichen oder wärmeempfindlichen Aufzeichnungsmaterialien.

Die neuen chromogenen Chinazolinverbindungen entsprechen der allgemeinen Formel

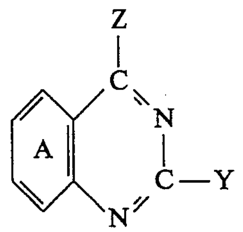

(1)

worin

Y einen aminosubstituierten Phenylrest der Formel

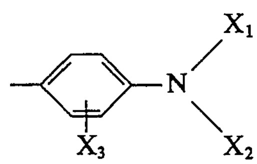

(1a)

oder einen 3-Carbazolylrest der Formel

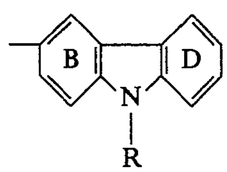

(1b)

und

Z Wasserstoff, $R_1$, $-OR_1'$, $-SR_1'$ oder $-NR_2R_3$ bedeuten, wobei

R Wasserstoff, unsubstituiertes oder durch Halogen, Hydroxy, Cyano oder Niederalkoxy substituiertes Alkyl mit höchstens 12 Kohlenstoffatomen, Alkenyl mit höchstens 12 Kohlenstoffatomen, Acyl mit 1 bis 12 Kohlenstoffatomen, Benzyl oder durch Halogen, Nitro, Niederalkyl, Niederalkoxy substituiertes Benzyl,

$R_1$ und $R_1'$ je unsubstituiertes oder durch Cyano oder Niederalkoxy substituiertes Alkyl mit höchstens 12 Kohlenstoffatomen, Cycloalkyl, unsubstituiertes oder substituiertes Aryl oder Aralkyl oder einen unsubstituierten oder substituierten heterocyclischen Rest und $R_1'$ auch Halogenalkyl mit 2 bis 6 Kohlenstoffatomen sein kann,

$R_2$, $R_3$, $X_1$ und $X_2$ unabhängig voneinander, Wasserstoff, unsubstituiertes oder durch Halogen, Hydroxy, Cyano oder Niederalkoxy substituiertes Alkyl mit höchstens 12 Kohlenstoffatomen, Cycloalkyl, Phenyl, Benzyl oder durch Halogen, Nitro, Cyano, Niederalkyl, Niederalkoxy oder Niederalkoxycarbonyl substituiertes Phenyl oder Benzyl und $R_2$ auch Acyl mit 1 bis 12 Kohlenstoffatomen, oder die Substituentenpaare ($R_2$ und $R_3$) und ($X_1$ und $X_2$), unabhängig voneinander, je zusammen mit dem sie verbindenden Stickstoffatom einen 5- oder 6gliedrigen heterocyclischen Rest,

$X_3$ Wasserstoff, Halogen, Nitro, Niederalkyl oder Niederalkoxy bedeuten und die Ringe A, B und D, unabhängig voneinander, unsubstituiert oder durch Cyano, Nitro, Halogen, Niederalkyl, Niederalkoxy oder Niederalkoxycarbonyl substituiert sein können und der Ring D auch einen unsubstituierten oder substituierten Phenylrest oder ankondensierten Benzolring aufweisen kann.

Niederalkyl und Niederalkoxy stellen bei der Definition der Reste der Chinazolinverbindungen solche Gruppen oder Gruppenbestandteile dar, die 1 bis 5, insbesondere 1 bis 3 Kohlenstoffatome aufweisen, wie z. B. Methyl, Äthyl, n-Propyl, Isopropyl, n-Butyl, sek.-Butyl, tert.-Butyl, oder Amyl bzw. Methoxy, Äthoxy oder Isopropoxy.

Der Ausdruck Aryl umfaßt bevorzugt Phenyl. »Acyl« ist besonders Formyl, Niederalkylcarbonyl, wie z. B. Acetyl oder Propionyl, oder Benzoyl. Weitere Acylreste sind Niederalkylsulfonyl, wie z. B. Methylsulfonyl oder Äthylsulfonyl sowie Phenylsulfonyl, Phenyl, Benzoyl und Phenylsulfonyl können durch Halogen, Methyl, Methoxy oder Äthoxy substituiert sein.

Y ist vorzugsweise ein aminosubstituierter Phenylrest der Formel (1a).

Stellen die Substituenten R, $R_1$, $R_1'$, $R_2$, $R_3$, $X_1$ und $X_2$ Alkylgruppen dar, so können sie geradkettig

oder verzweigt sein. Beispiele für solche Alkylreste sind Methyl, Äthyl, n-Propyl, Isopropyl, n-Butyl, sek.-Butyl, n-Hexyl, n-Octyl oder n-Dodecyl.

Sind die Alkylreste in R, $R_1$, $R_1'$, $R_2$, $R_3$, $X_1$ und $X_2$ substituiert, so handelt es sich vor allem um Cyanoalkyl oder Alkoxyalkyl, jeweils mit insgesamt 2 bis 6 Kohlenstoffatomen, wie z. B. $\beta$-Cyanoäthyl, $\beta$-Methoxyäthyl oder $\beta$-Äthoxyäthyl. Als Halogenalkyl kann $R_1'$ beispielsweise $\gamma$-Chlorpropyl oder vorzugsweise $\beta$-Chloroäthyl sein.

Alkenyl in R steht beispielsweise für Allyl, 2-Methallyl, 2-Äthallyl, 2-Butenyl oder Octenyl.

Der Acylrest in R und $R_2$ ist beispielsweise Formyl, Niederalkylcarbonyl oder Benzoyl, vorzugsweise jedoch Acetyl oder Propionyl. Benzoyl kann im Benzolring durch Halogen, Methyl oder Methoxy substituiert sein.

Beispiele für Cycloalkyl in der Bedeutung von $R_1$, $R_1'$, $R_2$, $R_3$, $X_1$ und $X_2$ sind Cyclopentyl oder vorzugsweise Cyclohexyl.

$R_1$ und $R_1'$ in der Bedeutung von Aralkyl stehen in der Regel für Phenyläthyl oder in erster Linie für Benzyl, während Aryl zweckmäßigerweise Naphthyl, Diphenyl und vor allem Phenyl bedeutet. Die Aralkyl- und Arylreste können durch Halogen, Nitro, Niederalkyl, Niederalkoxy, Niederalkoxycarbonyl oder Niederalkylcarbonyl substituiert sein.

Bevorzugte Substituenten in der Benzyl- und Phenylgruppe der $R_1'$, $R_1$-, $R_2$-, $R_3$-, $X_1$- und $X_2$-Reste und in der Benzylgruppe der R-Reste sind z. B. Halogene, Cyano, Nitro, Methyl, Methoxy oder Carbomethoxy. Beispiele für derartige araliphatische bzw. aromatische Reste sind Methylbenzyl, Chlorbenzyl, Nitrophenyl, Cyanophenyl, Tolyl, Xylyl, Chlorphenyl, Methoxyphenyl oder Carbomethoxyphenyl.

Als heterocyclischer Rest stellen $R_1$ und $R_1'$ in erster Linie einen fünf- oder sechsgliedrigen, vorzugsweise Sauerstoff, Schwefel oder Stickstoff aufweisenden Heterocyclus von aromatischem Charakter dar. Beispiele derartiger Heterocyclen sind Thienyl-, Furyl-, Pyrrolyl-, Pyrazolyl-, Imidazolyl- oder vorzugsweise Pyridylreste. Diese heterocyclischen Reste können substituiert sein, insbesondere durch Halogen, Cyano, Nitro, Niederalkyl, Niederalkoxy oder Niederalkoxycarbonyl. Die bevorzugten heterocyclischen Reste in der Bedeutung von $R_1$ und $R_1'$ sind 2-Furyl, 2-Thienyl und besonders 2-, 3- oder 4-Pyridyl.

Wenn die Substituentenpaare ($R_2$ und $R_3$) und ($X_1$ und $X_2$) zusammen mit dem gemeinsamen Stickstoffatom einen heterocyclischen Rest darstellen, so ist dieser beispielsweise Pyrrolidino, Piperidino, Pipecolino, Morpholino, Thiomorpholino oder Piperazino.

$X_1$ und $X_2$ bedeuten unabhängig voneinander vorzugsweise Niederalkyl, Benzyl, Phenyl oder Niederalkoxyphenyl. $X_3$ bedeutet vorzugsweise Wasserstoff, Methyl, Methoxy oder Chlor. R ist vorzugsweise $C_1-C_8$-Alkyl oder Benzyl und insbesondere Äthyl oder Butyl.

Die Ringe A, B und D sind vorzugsweise nicht weiter substituiert. Falls sie Substituenten aufweisen, sind sie unabhängig voneinander in erster Linie durch Halogen, Niederalkyl oder Niederalkoxy, z. B. durch Chlor, Methyl oder Methoxy weitersubstituiert. Pro Benzolring können vorteilhafterweise 1 oder 2 Substituenten vorhanden sein. Ein allfälliger Substituent des Ringes D befindet sich vorzugsweise in p-Stellung zum Stickstoff. Der Ring D kann auch einen oder zwei ankondensierte Benzolkerne enthalten, die somit z. B. einen 1,2-Benzocarbazol-, 3,4-Benzocarbazol- oder 1,2-, 3,4-Dibenzocarbazolring ergänzen.

Praktisch wichtige chromogene Chinazolinverbindungen entsprechen der Formel (1), wobei
R unsubstituiertes oder durch Halogen, Cyano oder Niederalkoxy substituiertes Alkyl mit höchstens 12 Kohlenstoffatomen, Niederalkylcarbonyl oder unsubstituiertes oder durch Halogen, Niederalkyl oder Niederalkoxy substituiertes Benzyl,
$R_1$ und $R_1'$ je unsubstituiertes oder durch Niederalkoxy substituiertes Alkyl mit höchstens 8 Kohlenstoffatomen, Cyclohexyl, Phenyl, Naphthyl, Benzyl oder durch Halogen, Nitro, Cyano, Niederalkyl, Niederalkoxy oder Niederalkoxycarbonyl substituiertes Phenyl oder Benzyl,
$R_2$ und $R_3$ unabhängig voneinander Wasserstoff, Niederalkyl, Phenyl, Niederalkylphenyl, Niederalkoxyphenyl oder Benzyl, und $R_2$ auch Niederalkylcarbonyl, Niederalkylsulfonyl, Benzoyl oder Phenylsulfonyl,
$X_1$ und $X_2$ unabhängig voneinander Niederalkyl, Phenyl, Niederalkylphenyl, Niederalkoxyphenyl oder Benzyl und $X_1$ auch Wasserstoff, oder die Substituentenpaare
($R_2$ und $R_3$) und ($X_1$ und $X_2$), unabhängig voneinander, je zusammen mit dem sie verbindenden Stickstoffatom, Pyrrolidino, Piperidino, oder Morpholino, und
$X_3$ Wasserstoff, Halogen, Niederalkyl oder Niederalkoxy bedeuten und die Ringe A, B und D unabhängig voneinander, unsubstituiert oder durch Cyano, Halogen, Niederalkyl oder Niederalkoxy substituiert sein können und der Ring D auch einen oder zwei ankondensierte Benzolringe enthalten kann.

Unter den Chinazolinverbindungen der Formel (1) sind diejenigen, in denen Y ein aminosubstituierter Phenylrest der Formel (1a) ist, bevorzugt. Z bedeutet vorzugsweise $-OR_1'$. $R_1'$ ist vorzugsweise Niederalkyl oder Phenyl. Vorteilhafterweise ist $R_1'$ auch Pyridyl.

Von großem Interesse sind Chinazolinverbindungen der Formeln

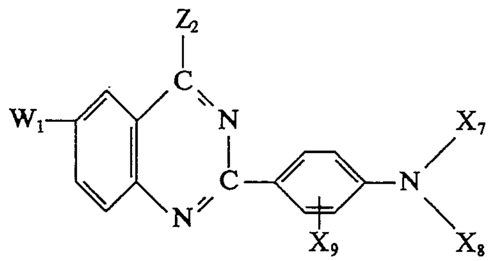

(3)

oder

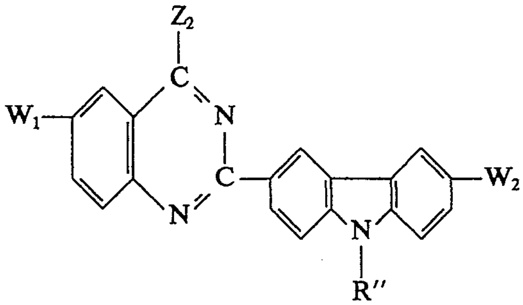

(4)

worin $Z_2$ Wasserstoff, $R_7$, $-OR_7$, $-SR_7$ oder $-NR_8R_9$,
$R_7$ Niederalkyl, Niederalkoxy-niederalkyl, Cyclohexyl, Phenyl, Naphthyl, Benzyl oder durch Halogen, Cyano, Nitro, Methyl oder Methoxy substituiertes Phenyl,
$R_8$ und $R_9$ unabhängig voneinander, Wasserstoff, Niederalkyl, Phenyl oder Benzyl und $R_8$ auch Niederalkylcarbonyl oder Benzoyl,
$X_7$ Niederalkyl, Phenyl, Niederalkylphenyl, Niederalkoxyphenyl oder Benzyl,
$X_8$ Wasserstoff, Niederalkyl, Phenyl oder Benzyl,
$X_9$ Wasserstoff, Methyl, Methoxy oder Äthoxy,
R'' Alkyl mit 1 bis 8 Kohlenstoffatomen oder Benzyl und
$W_1$ und $W_2$ unabhängig voneinander, Halogen, Methoxy, Methyl oder besonders Wasserstoff bedeuten.

Im Vordergrund des Interesses stehen Chinazolinverbindungen der Formel (3), in der $Z_2$ $-OR_7$ bedeutet. $-OR_7$ ist vorzugsweise Niederalkoxy oder Phenoxy.

Halogen in Verbindung mit den vorstehenden Substituenten in Formeln (1) bis (4) bedeutet beispielsweise Fluor, Brom oder vorzugsweise Chlor.

Die erfindungsgemäßen Chinazolinverbindungen der Formel (1), in der Z $-OR_1'$, $-SR_1'$ oder $-NR_2R_3$ bedeutet, werden dadurch hergestellt, daß man eine 4-Halogenchinazolinverbindung der Formel

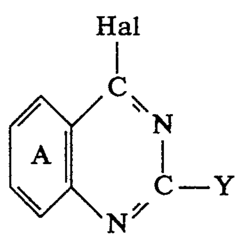

(5)

worin A und Y die angegebene Bedeutung haben und Hal Halogen, wie z. B. Brom, Fluor oder vorzugsweise Chlor bedeutet, mit einer Verbindung der Formeln

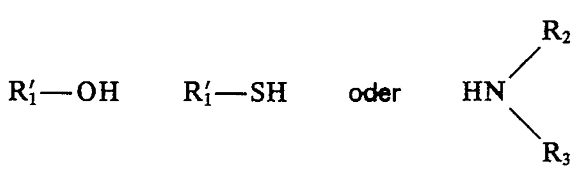

(6a) (6b) oder (6c)

umsetzt, worin $R_1'$, $R_2$ und $R_3$ die angegebene Bedeutung haben.

Die Umsetzung der Verbindung der Formel (5) mit einer Verbindung der Formel (6a), (6b) oder (6c)

erfolgt zweckmäßig in Anwesenheit eines säurebindenden Mittels, wie z. B. eines Alkalimetallcarbonates oder einer tertiären Stickstoffbase, wie Pyridin oder Trialkylaminen, gegebenenfalls in einem organischen Lösungsmittel und bei Rückflußtemperatur.

Als Lösungsmittel kommen beispielsweise cycloaliphatische oder aromatische Kohlenwasserstoffe, wie z. B. Cyclohexan, Benzol, Toluol oder Xylol; Chlorkohlenwasserstoffe, wie Chloroform, Äthylenchlorid oder Chlorbenzole; Äther, wie Diäthyläther oder Glykoldimethyläther; cyclische Äther, wie Dioxan oder Tetrahydrofuran; sowie Dimethylformamid, Diäthylformamid, Dimethylsulfoxid oder Acetonitril in Betracht.

Eine Ausführungsform zur Herstellung von Verbindungen der Formel (1), in der Z Wasserstoff bedeutet, besteht darin, daß man die 4-Halogen-chinazolinverbindung der Formel (5) alkalisch enthalogeniert, wobei das Halogenatom durch Wasserstoff ersetzt wird. Diese Enthalogenierung erfolgt beispielsweise gemäß J. Chem. Soc. 1962, 561—572 unter Verwendung von Toluol-p-sulphonylhydrazid und Zersetzung des entstehenden 4-(N-Toluol-p-sulphonylhydrazino-)chinazolins mit Alkalien, z. B. Natriumhydroxyd, vorzugsweise in Äthylenglykol oder Äthylenglykolmonomethyläther.

Die Ausgangsstoffe der Formel (5) können dadurch hergestellt werden, daß man beispielsweise ein 2-Amino-benzoesäureamid der Formel

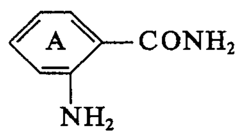

(7)

mit einem Aldehyd der Formel

Y—CHO (8)

zu einer 1,2,3,4-Tetrahydro-Chinazolinon(4)-Verbindung der Formel

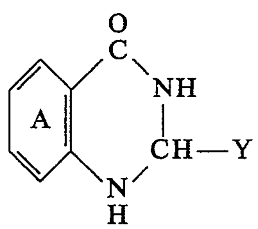

(9)

umsetzt, diese zu einer Verbindung der Formel

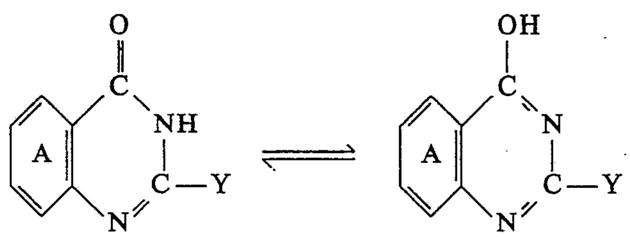

(10)

oxidiert, sodann die Hydroxylgruppe am heterocyclischen Ring des Chinazolinsystems durch ein Halogenatom, z. B. mittels Thionylchlorid in Dimethylformamid unter Bildung des Ausgangsstoffes der Formel (5) ersetzt.

Die Oxidation der Umsetzungsprodukte der Formel (9) zu den 4-Chinazolonverbindungen der Formel (10) erfolgt mit Oxidationsmitteln. Geeignete Oxidationsmittel sind z. B. Chromate, Bichromate, Chlorate, Chlorite, Peroxide, z. B. Wasserstoffperoxid, Mangandioxid, Bleidioxid, molekularer Sauerstoff, Luft, Perborate, Permanganate, Chlor, Brom und vor allem Chloranil.

Die besten Ergebnisse in bezug auf Ausbeute und Reinheit der erhaltenen 4-Chinazolonverbindungen erzielt man mit Chloranil als bevorzugtes Oxidationsmittel, das vorzugsweise in Dimethylformamid angewandt wird.

Ein bevorzugtes Verfahren zur Herstellung von Verbindungen der Formel (1), in der Z $R_1$ bedeutet, besteht darin, daß man eine Ketoamidoverbindung der Formel

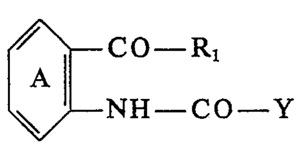

(11)

worin A, $R_1$ und Y die angegebene Bedeutung haben, mit einer alkoholischen, vorzugsweise methanolischen Ammoniaklösung zu einer Chinazolinverbindung der Formel

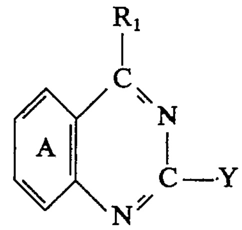

(12)

reagieren läßt. Die Umsetzung der Ketoamidoverbindung der Formel (11) mit der Ammoniaklösung kann bei einer Temperatur von 80 bis 200°C, vorzugsweise 100 bis 180°C, vorgenommen werden. Verbindungen der Formel (11) können gemäß A. Bischler und D. Barad, Ber., 25, 3080 (1892) und A. Bischler und F. J. Howell, Ber. 26, 1384 (1893) durch Acylierung der entsprechenden Ketoaminoverbindungen mit den gewünschten Säureanhydriden oder Säurehalogeniden hergestellt werden.

Die Chinazolinverbindungen der Formeln (1) bis (4) sind normalerweise farblos oder schwach gefärbt. Wenn diese Farbbildner mit einem vorzugsweise sauren Entwickler, z. B. einem Elektronenakzeptor, in Kontakt gebracht werden, so ergeben sie je nach Bedeutung von Y und Z intensive, gelbe, orange oder rote Farbtöne, die ausgezeichnet sublimations- und lichtecht sind. Sie sind deshalb auch sehr wertvoll im Gemisch mit einem oder mehreren anderen bekannten Farbbildnern, z. B. 3,3-(Bis-aminophenyl-)phthaliden, 3,3-(Bis-indolyl-)phthaliden, 3-Aminofluoranen, 2,6-Diaminofluoranen, Leukoauramine, Spiropyranen, Phenoxazinen, Phenothiazinen oder Triarylmethan-leukofarbstoffen, um blaue, marineblaue, graue oder schwarze Färbungen zu ergeben.

Die Chinazolinverbindungen der Formeln (1) bis (4) zeigen sowohl auf phenolischen Unterlagen, wie auch besonders auf Tonen, eine verbesserte Farbintensität und Lichtechtheit. Sie eignen sich vor allem als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann.

Ein druckempfindliches Material besteht beispielsweise aus mindestens einem Paar von Blättern, die mindestens einen Farbbildner der Formeln (1) bis (4) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z. B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Kaolin oder irgendein beliebiger Ton oder sauer reagierende, organische Verbindung, wie z. B. gegebenenfalls ringsubstituierte Phenole, Salicylsäure oder Salicylsäureester und deren Metallsalze, ferner ein sauer reagierendes, polymeres Material, wie z. B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Äthylen oder Vinylmethyläther, oder Carboxypolymethylen. Es können auch Mischungen der genannten polymeren Verbindungen eingesetzt werden. Bevorzugte Entwickler sind säureaktiviertes Bentonit, Zinksalicylate oder die Kondensationsprodukte von p-substituierten Phenolen mit Formaldehyd. Die letzteren können auch Zink enthalten.

Die Entwickler können zusätzlich auch mit anderen, an sich unreaktiven oder wenig reaktiven Pigmenten gemischt eingesetzt werden. Beispiele für solche Pigmente sind: Talk, Titandioxid, Zinkoxid, Kreide, Tone wie Kaolin, sowie organische Pigmente, z. B. Harnstoff-Formaldehyd oder Melamin-Formaldehyd-Kondensationsprodukte.

Der Farbbildner liefert an den Punkten, an denen er mit dem Elektronenakzeptor in Kontakt kommt, eine gefärbte Markierung. Um zu verhindern, daß die Farbbildner, die in dem druckempfindlichen Aufzeichnungsmaterial enthalten sind, frühzeitig aktiv werden, werden sie in der Regel von dem Elektronenakzeptor getrennt. Dies kann zweckmäßig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Wenn die Kapseln durch Druck, beispielsweise mittels eines Bleistiftes zerbrochen werden und wenn die Farbbildnerlösung auf diese Weise auf ein benachbartes Blatt übertragen wird, das mit einem Elektronenakzeptor beschichtet ist, wird eine farbige Stelle erzeugt. Diese Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z. B. polyhalogeniertes Paraffin oder Diphenyl, wie Chlorparaffin oder Trichlordiphenyl, ferner Tricresylphosphat, Di-n-butylphthalat, Dioctylphthalat, Trichlorbenzol, Trichloräthylphosphat, aromatische Äther, wie Benzylphenyläther, Kohlenwasserstofföle, wie Paraffin oder Kerosin, alkylierte

Derivate von Diphenyl, Naphthalin oder Triphenyl, Dibenzyltoluol, Terphenyl, partiell hydriertes Terphenyl, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und partiell hydriertem Terphenyl, eingesetzt, um eine optimale Löslichkeit für die Farbbildung, eine rasche und intensive Färbung und eine für die Mikroverkapselung günstige Viskosität zu erreichen.

Die Kapselwände können durch Koazervationskräfte gleichmäßig um die Tröpfchen der Farbbildnerlösung herum gebildet werden, wobei das Einkapselungsmaterial z. B. aus Gelatine und Gummiarabikum bestehen kann, wie dies z. B. in der US-Patentschrift 2 800 457 beschrieben ist. Die Kapseln können vorzugsweise auch aus einem Aminoplast oder modifizierten Aminoplasten durch Polykondensation gebildet werden, wie es in den britischen Patentschriften 989 264, 1 156 725, 1 301 052 und 1 355 124 beschrieben ist. Ebenfalls geeignet sind Mikrokapseln, welche durch Grenzflächenpolymerisation gebildet werden, wie z. B. Kapseln aus Polyester, Polycarbonat, Polysulfonamid, Polysulfonat, besonders aber aus Polyamid oder Polyurethan.

Die Farbbildner der Formel (1) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Übertragungsblattes und der Elektronenakzeptor in Form einer Schicht auf der Vorderseite eines Empfangsblattes vorhanden sind.

Eine andere Anordnung der Bestandteile besteht darin, daß die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummiarabikum, Polyvinylalkohol, Hydroxymethylcellulose, Casein, Methylcellulose, Dextrin, Stärke oder Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (1) bis (4) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel.

Thermoreaktive Aufzeichnungssysteme umfassen z. B. wärmeempfindliche Aufzeichnungs- und Kopiermaterialien und -papiere. Diese Systeme werden beispielsweise zum Aufzeichnen von Informationen, z. B. in elektronischen Rechnern, Ferndruckern, Fernschreibern oder in Aufzeichnungsgeräten und Meßinstrumenten, verwendet. Die Bilderzeugung (Markierungserzeugung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, daß der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, daß sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-B-1 251 348 beschrieben sind, z. B. 4-tert.-Butylphenol, 4-Phenylphenol, 4-Hydroxydiphenyläther, α-Naphthol, β-Naphthol, 4-Hydroxybenzoesäuremethylester, 4-Hydroxyacetophenon, 2,2'-Dihydroxydiphenyl, 4,4'-Isopropylidendiphenol, 4,4'-Isopropyliden-bis-(2-methylphenol), 4,4'-Bis-(hydroxyphenyl)valeriansäure, Hydrochinon, Pyrogallol, Phloroglucin, p-, m-, o-Hydroxybenzoesäure, Gallussäure, 1-Hydroxy-2-naphthoesäure sowie Borsäure und organische, vorzugsweise aliphatische Dicarbonsäuren, wie z. B. Weinsäure, Oxalsäure, Maleinsäure, Zitronensäure, Citraconsäure und Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Chinazolinverbindungen und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so daß der Farbbildner mit dem Entwickler in Kontakt kommt und sich eine Farbe bilden kann. Wasserlösliche oder mindestens in Wasser quellbare Bindemittel sind z. B. hydrophile Polymerisate, wie Polyvinylalkohol, Polyacrylsäure,

Hydroxyäthylcellulose, Methylcellulose, Carboxymethylcellulose, Polyacrylamid, Polyvinylpyrrolidon, Gelatine und Stärke.

Wenn der Farbbildner und der Entwickler in zwei getrennten Schichten vorliegen, können in Wasser unlösliche Bindemittel, d. h. in nicht-polaren oder nur schwach polaren Lösungsmitteln lösliche Bindemittel, wie z. B. Naturkautschuk, synthetischer Kautschuk, chlorierter Kautschuk, Alkylharze, Polystyrol, Styrol/Butadien-Mischpolymerisate, Polymethylacrylate, Äthylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weißgrades, zur Erleichterung des Bedruckens der Papiere und zur Verhinderung des Festklebens der erhitzten Feder können diese Schichten, z. B. Talk, Titandioxyd, Zinkoxyd, Calciumcarbonat, Tone oder auch organische Pigmente, wie z. B. Harnstoff-Formaldehydpolymerisate enthalten. Um zu bewirken, daß nur innerhalb eines begrenzten Temperaturbereiches die Farbe gebildet wird, können Substanzen, wie Harnstoff, Thioharnstoff, Acetamid, Acetanilid, Stearinsäureamid, Phthalsäureanhydrid, Metallstearate, Phthalsäurenitril oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z. B. Carnaubawachs, Paraffinwachs, Polyäthylenwachs.

In den folgenden Beispielen beziehen sich die angegebenen Prozentsätze, wenn nichts anderes angegeben ist, auf das Gewicht.

Beispiel 1

Zu einer Lösung von 1,35 g Natriummethylat in 50 ml Methanol werden 2,8 g 4-Chlor-2-(4'-dimethylaminophenyl-)chinazolin zugegeben. Alsdann wird die erhaltene Suspension während 2$^1/_2$ Stunden am Rückfluß gerührt und auf 5°C abgekühlt, worauf das ausgeschiedene Produkt abfiltriert, mit Wasser gewaschen und getrocknet wird. Nach anschließender Behandlung des Produktes mit 20 ml kaltem Toluol, Eindampfen der Toluollösung und Umkristallisation des Rückstandes aus Methanol erhält man 1,4 g einer Verbindung der Formel

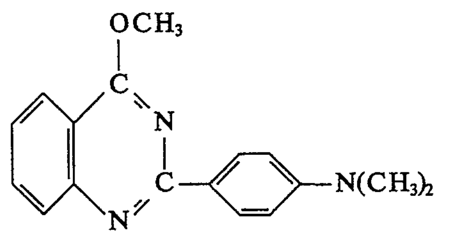

(21)

mit einem Schmelzpunkt von 94—95° C.

Auf Säureton entwickelt dieser Farbbildner eine gelbe Farbe.

Das im Beispiel 1 verwendete 4-Chlor-2-(4'-dimethylaminophenyl-)chinazolin kann wie folgt hergestellt werden:

13,6 g Anthranilsäureamid und 14,9 g 4-Dimethylamino-benzaldehyd werden in 200 ml Äthanol während 2 Tagen am Rückfluß gerührt. Nach Abkühlung auf 20°C wird das ausgefallene Produkt abfiltriert, mit Äthanol gewaschen und getrocknet. Man erhält 21,8 g einer Verbindung der Formel

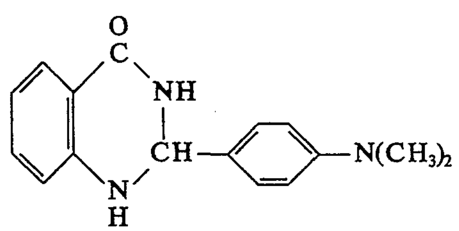

(i)

mit einem Schmelzpunkt von 214—217° C.

13,4 g der Verbindung der Formel (i) werden bei 50°C in 100 ml Dimethylformamid gelöst. Alsdann läßt man die Lösung zu einer auf 50°C vorgewärmten Suspension von 12,4 g Chloranil in 150 ml Dimethylformamid bei 50—55°C zutropfen. Danach wird das Reaktionsgemisch 3 Stunden weitergerührt, dann auf 10°C abgekühlt. Hierauf wird das auskristallisierte Produkt abfiltriert, mit Äthanol gewaschen und getrocknet. Man erhält 10,4 g einer Verbindung der Formel

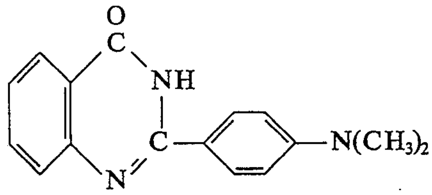
(ii)

mit einem Schmelzpunkt von 260—262° C.

5,3 g der Verbindung der Formel (ii) werden in 30 ml Dimethylformamid suspendiert und unter Rühren mit 2,5 ml Thionylchlorid versetzt. Hierauf läßt man nach 1 1/2stündigem Rühren bei Raumtemperatur 50 ml Methanol und 5 ml Ammoniakwasser (80%) zutropfen. Man kühlt das Reaktionsgemisch auf 20° C ab, filtriert den Niederschlag ab, wäscht mit Methanol und trocknet. Man erhält 3,5 g 4-Chlor-2-(4'-dimethylaminophenyl-)chinazolin der Formel

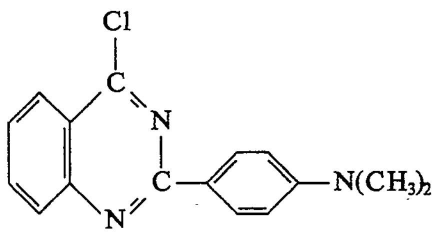
(iii)

mit einem Schmelzpunkt von 167—170° C.

## Beispiel 2

2,8 g 4-Chlor-2-(4'-dimethylaminophenyl-)chinazolin werden zusammen mit 15 g Phenol und 2,1 g wasserfreiem Kaliumcarbonat während 45 Minuten bei 145° C gerührt. Nach Abkühlung auf 60° C läßt man in das Reaktionsgemisch 80 ml einer 2 N-Natriumhydroxydlösung zutropfen und rührt weiter während 30 Minuten. Hierauf wird das ausgefalllene Produkt abfiltriert, mit Wasser gewaschen und getrocknet. Nach Umkristallisieren des Produktes aus Äthanol erhält man 1,4 g einer Verbindung der Formel

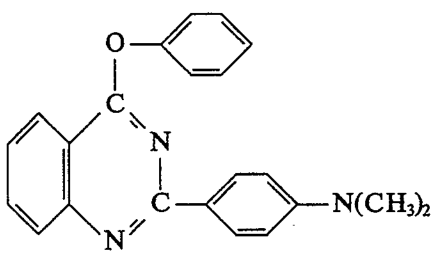
(22)

mit einem Schmelzpunkt von 187–189°C.

Auf Säureton entwickelt dieser Farbbildner eine gelbe Farbe.

Auf gleiche Weise wie im Beispiel 1 oder 2 beschrieben, erhält man unter Verwendung der entsprechenden Ausgangsstoffe die in der folgenden Tabelle aufgeführten Farbbildner der Formel

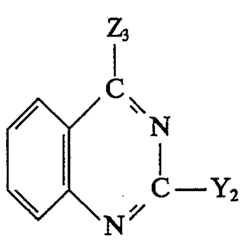
(23)

Tabelle

| Beispiel Nr. | $Z_3$ | $Y_2$ | Smp./°C | Farbe auf Säureton |
|---|---|---|---|---|
| 3 | $-OCH_2CH_2OCH_3$ | 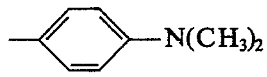 | 89–93 | gelb |
| 4 | $-OC(CH_3)_3$ | 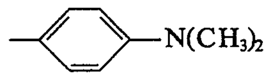 | 127–130 | gelb |
| 5 | 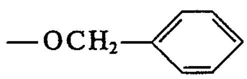 | 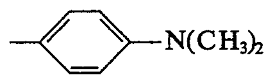 | 114–115 | gelb |
| 6 | 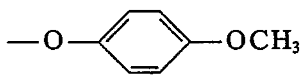 | 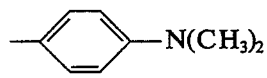 | 167–168 | gelb |
| 7 | 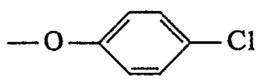 | 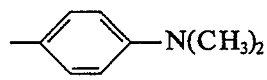 | 146–148 | gelb |
| 8 | 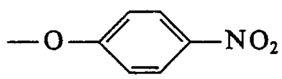 | 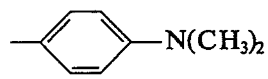 | 175–176 | orange |
| 9 | 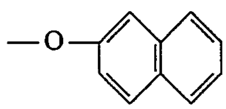 | 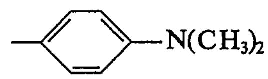 | 184–185 | gelb |
| 10 | 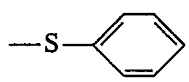 | 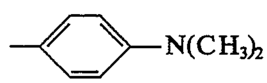 | 162–164 | orange |
| 11 | 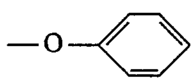 | 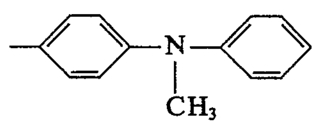 | 49–51 | gelb |
| 12 | 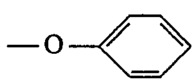 | 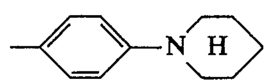 | 118–120 | gelb |
| 13 | 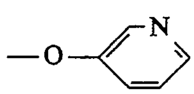 | 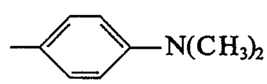 | 158–159 | orange |
| 14 | 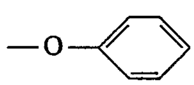 | 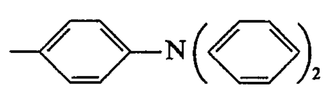 | 79–82 | gelb |
| 15 | 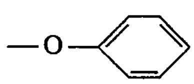 | 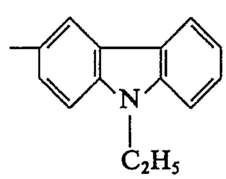 | 210–211 | gelb |

Beispiel 16

2,8 g 4-Chlor-2-(4′-dimethylaminophenyl-)chinazolin werden in 25 ml Dimethylformamid bei 75°C gelöst. Hierauf wird die Lösung bei 65°C mit 2,5 g einer 40%igen wäßrigen Dimethylaminlösung versetzt. Das Reaktionsgemisch wird während 2 Stunden gerührt, mit 25 ml Äthanol versetzt und auf 5°C abgekühlt, worauf das ausgeschiedene Produkt abfiltriert, mit Äthanol gewaschen und getrocknet wird. Man erhält 1,9 g einer Verbindung der Formel

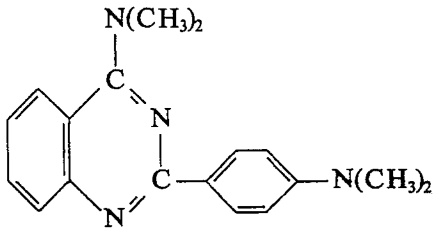

(24)

mit einem Schmelzpunkt von 154–156°C.

Auf Säureton entwickelt dieser Farbbildner eine gelbe Farbe.

Beispiel 17

Ersetzt man im Beispiel 16 das Dimethylamin durch 2,2 g N-Methylanilin und verfährt im übrigen wie im Beispiel 16 beschrieben, so werden 0,8 g einer Verbindung der Formel

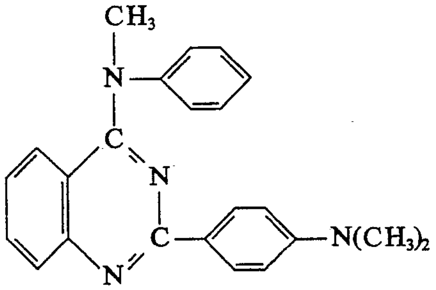

(25)

mit einem Schmelzpunkt von 165–167°C erhalten.

Auf Säureton entwickelt dieser Farbbildner eine gelbe Farbe.

Beispiel 18

Ersetzt man im Beispiel 16 das Dimethylamin durch 0,9 g Morpholin und verfährt im übrigen wie im Beispiel 16 beschrieben, so werden 2,0 g einer Verbindung der Formel

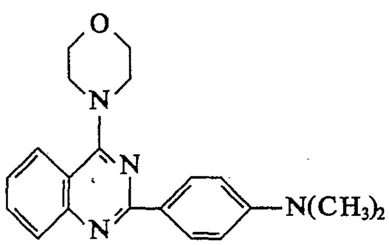

(26)

mit einem Schmelzpunkt von 180—184°C erhalten. Auf Säureton entwickelt dieser Farbbildner eine gelbe Farbe.

Beispiel 19

2,8 g 4-Chlor-2-(4′-dimethylaminophenyl-)chinazolin werden in 25 ml Chloroform bei 40°C gelöst. Diese Lösung wird auf 20°C abgekühlt und mit einer Lösung von 1,9 g Toluol-4-sulfonsäurehydrazid in 30 ml Chloroform versetzt. Die Reaktionslösung wird 18 Stunden unter Rückfluß gerührt und auf 10°C abgekühlt, worauf das ausgeschiedene Produkt abfiltriert, mit Chloroform gewaschen und getrocknet wird. Alsdann läßt man 25 ml einer 1N Natriumhydroxylösung in die Suspension des Produktes in 100 ml 2-Methoxyäthanol bei 90°C zutropfen.

Nach einer Stunde bei 95°C wird die Suspension auf 20°C abgekühlt, worauf der Niederschlag durch Abfiltrieren entfernt wird. Das Filtrat wird eingedampft und der Rückstand mit Methylenchlorid extrahiert. Nach Chromatographie und Umkristallisation aus Äthanol erhält man 0,3 g einer Verbindung der Formel

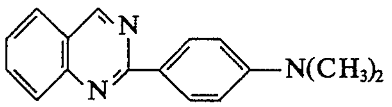

(27)

mit einem Schmelzpunkt von 136—137° C.
Auf Säureton entwickelt dieser Farbbildner eine orange Farbe.

Beispiel 20

9,5 g 4-Diäthylaminobenzoesäurechlorid und 8,85 g 2-Aminobenzophenon werden in 100 ml Toluol gelöst, worauf die Lösung 5 Stunden unter Rückfluß gerührt wird. Hierauf wird das Toluol durch Eindampfen entfernt. Der Rückstand wird danach in heißem Methanol gelöst und die Lösung auf 0° C abgekühlt. Die ausgeschiedenen Kristalle werden schließlich abfiltriert und getrocknet. Man erhält 11,6 g einer Verbindung der Formel

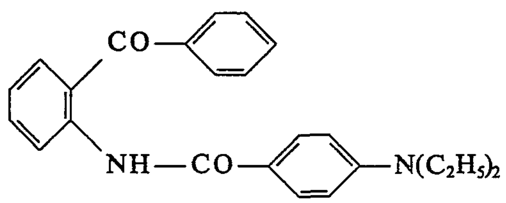

(iv)

mit einem Schmelzpunkt von 138 bis 140° C.

11,5 g der Verbindung der Formel (iv) werden zu 45 g einer 10%igen methanolischen Ammoniak-Lösung gegeben, worauf die Mischung in einem Autoklaven während 5 Stunden bei 150° C gerührt wird. Nach Abkühlen auf Raumtemperatur wird das Reaktionsprodukt zur Trockene eingedampft und der Rückstand aus Ligroin umkristallisiert. Man erhält nach dem Trocknen der Kristalle 7,4 g einer Verbindung der Formel

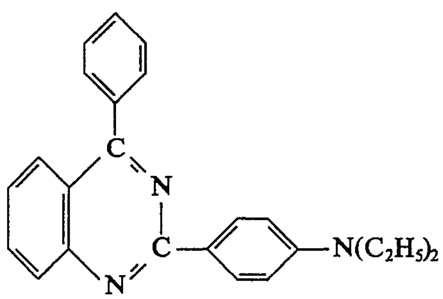

(28)

mit einem Schmelzpunkt von 132 bis 134°C. Auf Säureton entwickelt dieser Farbbildner eine rote Farbe.

Beispiel 21

Ersetzt man im Beispiel 20 das 2-Aminobenzophenon durch 6,1 g 2-Aminoacetophenon und verfährt im übrigen wie im Beispiel 20 beschrieben, so erhält man 5,4 g einer Verbindung der Formel

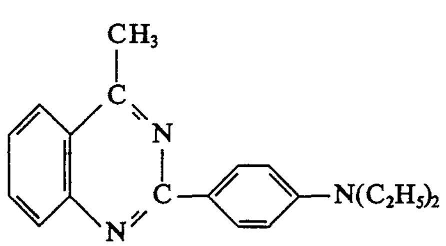

(29)

mit einem Schmelzpunkt von 138 bis 139° C.
Auf Säureton entwickelt dieser Farbbildner eine orange Farbe.

Beispiel 22

Herstellung eines druckempfindlichen Kopierpapiers

Eine Lösung von 3 g der Chinazolinverbindung der Formel (22) in 80 g partiell hydriertem Terphenyl und 17 g Kerosin wird auf an sich bekannte Weise mit Gelatine und Gummiarabikum durch Koazervation mikroverkapselt, mit Stärkelösung vermischt und auf ein Blatt Papier gestrichen. Ein zweites Blatt Papier wird auf der Frontseite mit säureaktiviertem Bentonit als Farbentwickler beschichtet. Das erste Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive gelbe Kopie, die ausgezeichnet lichtecht ist.

Entsprechende intensive und lichtechte gelbe, orange bzw. rote Kopien werden auch bei Verwendung jedes der anderen in den Herstellungsbeispielen angegebenen Farbbildner der Formeln (21) und (23) bis (29) erzielt.

Beispiel 23

Ersetzt man in Beispiel 22 die Chinazolinverbindung der Formel (22) durch eine Mischung der folgenden Zusammensetzung

1,2 g 3,3-Bis-(4'-dimethylaminophenyl)-6-dimethylaminophthalid,
1,2 g N-Butylcarbazol-3-yl-bis-(4'-N-methyl-N-phenylaminophenyl-)methan
0,4 g der Chinazolinverbindung der Formel (22) und
0,4 g 3,3-Bis-(N-n-octyl-2'-methylindol-3'-yl-)phthalid

und verfährt im übrigen wie in Beispiel 22 beschrieben, so erhält man ein druckempfindliches Aufzeichnungsmaterial, welches durch Schreiben mit der Hand oder mit der Schreibmaschine eine intensive und lichtechte schwarze Kopie ergibt.

Beispiel 24

1 g der Chinazolinverbindung der Formel (21) wird in 17 g Toluol gelöst. Zu dieser Lösung gibt man unter Rühren 12 g Polyvinylacetat, 8 g Calciumcarbonat und 2 g Titandioxyd. Die erhaltene Suspension wird im Gewichtsverhältnis 1 : 1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m$^2$ mit einer Mischung bestehend aus 1 Teil eines Amidwachses, 1 Teil eines Stearinwachses und 1 Teil Zinkchlorid beschichtet worden ist. Durch Schreiben mit der Hand oder mit der Schreibmaschine auf dem oberen Blatt wird Druck ausgeübt, und es entwickelt sich sofort auf dem mit dem Farbbildner beschichteten Blatt, eine intensive und lichtechte gelbe Farbe.

Beispiel 25

Herstellung eines wärmeempfindlichen Aufzeichnungsmaterials

In einer Kugelmühle werden 32 g 2,2-Bis-(4-hydroxyphenyl)-propan (Bisphenol A), 3,8 g Distearylamid des Äthylendiamins, 39 g Kaolin, 20 g eines zu 88% hydrolysierten Polyvinylalkohols und 500 ml Wasser gemahlen bis die Teilchengröße ca. 5 µm beträgt. In einer zweiten Kugelmühle werden 6 g der Verbindung der Formel (21), 3 g eines zu 88% hydrolysierten Polyvinylalkohols und 60 ml Wasser zu einer Teilchengröße von ca. 3 µm gemahlen.

Die beiden Dispersionen werden zusammengegeben und mit einem Trockenauftragsgewicht von 5,5 g/m$^2$ auf ein Papier gestrichen. Durch Berührung des Papiers mit einem erhitzten Kugelschreiber wird eine intensive gelbe Farbe erhalten, die eine ausgezeichnete Lichtechtheit hat.

Intensive und lichtechte gelbe, orange bzw. rote Farben können auch bei Verwendung jeder der anderen Farbbildner der Formeln (22) bis (29) erhalten werden.

Beispiel 26

In einer Kugelmühle werden 2,7 g der Chinazolinverbindung der Formel (22), 24 g N-Phenyl-N'-(1-hydroxy-2,2,2-trichloroäthyl)-harnstoff, 16 g Stearinsäureamid, 59 g eines zu 88% hydrolysierten Polyvinylalkohols und 58 ml Wasser gemahlen bis die Teilchengröße 2–5 µm beträgt. Diese

Suspension wird bei einem Trockenauftragsgewicht von 5,5 g/m² auf ein Blatt Papier gestrichen. Durch Berührung des Papiers mit einem erhitzten Kugelschreiber wird eine intensive und lichtechte gelbe Farbe erhalten.

**Patentansprüche**

1. Chromogene Chinazolinverbindungen der Formel

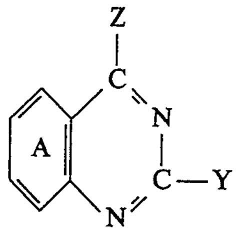

(1)

worin
Y einen aminosubstituierten Phenylrest der Formel

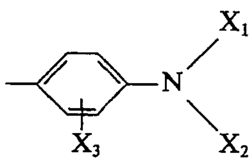

(1a)

oder einen 3-Carbazolylrest der Formel

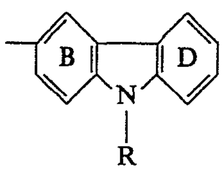

(1b)

und
Z Wasserstoff, $R_1$, $-OR_1'$, $-SR_1'$ oder $-NR_2R_3$ bedeuten, wobei
R Wasserstoff, unsubstituiertes oder durch Halogen, Hydroxy, Cyano oder Niederalkoxy substituiertes Alkyl mit höchstens 12 Kohlenstoffatomen, Alkenyl mit höchstens 12 Kohlenstoffatomen, Acyl mit 1 bis 12 Kohlenstoffatomen, Benzyl oder durch Halogen, Nitro, Niederalkyl, Niederalkoxy substituiertes Benzyl,
$R_1$ und $R_1'$ je unsubstituiertes oder durch Cyano oder Niederalkoxy substituiertes Alkyl mit höchstens 12 Kohlenstoffatomen, Cycloalkyl, unsubstituiertes oder substituiertes Aryl oder Aralkyl oder einen unsubstituierten oder substituierten heterocyclischen Rest und $R_1'$ auch Halogenalkyl mit 2 bis 6 Kohlenstoffatomen,
$R_2$, $R_3$, $X_1$ und $X_2$ unabhängig voneinander Wasserstoff, unsubstituiertes oder durch Halogen, Hydroxy, Cyano oder Niederalkoxy substituiertes Alkyl mit höchstens 12 Kohlenstoffatomen, Cycloalkyl, Phenyl, Benzyl oder durch Halogen, Nitro, Cyano, Niederalkyl, Niederalkoxy oder Niederalkoxycarbonyl substituiertes Phenyl oder Benzyl und $R_2$ auch Acyl mit 1 bis 12 Kohlenstoffatomen, oder die Substituentenpaare ($R_2$ und $R_3$) und ($X_1$ und $X_2$), unabhängig voneinander, je zusammen mit dem sie verbindenden Stickstoffatom einen 5- oder 6gliedrigen heterocyclischen Rest,
$X_3$ Wasserstoff, Halogen, Nitro, Niederalkyl oder Niederalkoxy bedeuten und die Ringe A, B und D, unabhängig voneinander, unsubstituiert oder durch Cyano, Nitro, Halogen, Niederalkyl, Niederalkoxy oder Niederalkoxycarbonyl substituiert sein können und der Ring D auch einen unsubstituierten oder substituierten Phenylrest oder ankondensierten Benzolring aufweisen kann, wobei der Ausdruck »nieder« sich auf solche Gruppen mit 1 bis 5 Kohlenstoffatome bezieht.

2. Chinazolinverbindungen gemäß Anspruch 1, dadurch gekennzeichnet, daß in Formel (1) $R_1$ und $R_1'$ je unsubstituiertes oder durch Cyano oder Niederalkoxy substituiertes Alkyl mit höchstens 12 Kohlenstoffatomen, Cycloalkyl, unsubstituiertes oder substituiertes Aryl oder Aralkyl und $R_1'$ auch Halogenalkyl mit 2 bis 6 Kohlenstoffatomen bedeuten.

3. Chinazolinverbindungen gemäß Anspruch 1, dadurch gekennzeichnet, daß in Formel (1) $R_1$ und $R_1'$ je einen fünf- oder sechsgliedrigen, heterocyclischen Rest aromatischen Charakters bedeuten, der unsubstituiert oder durch Halogen, Cyano, Nitro, Niederalkyl, Niederalkoxy oder Niederalkoxycarbonyl substituiert ist.

4. Chinazolinverbindungen gemäß Anspruch 1, dadurch gekennzeichnet, daß
R unsubstituiertes oder durch Halogen, Cyano oder Niederalkoxy substituiertes Alkyl mit höchstens 12 Kohlenstoffatomen, Niederalkylcarbonyl oder unsubstituiertes oder durch Halogen, Niederalkyl oder Niederalkoxy substituiertes Benzyl,
$R_1$ und $R_1'$ je unsubstituiertes oder durch Niederalkoxy substituiertes Alkyl mit höchstens 8 Kohlenstoffatomen, Cyclohexyl, Phenyl, Naphthyl, Benzyl oder durch Halogen, Nitro, Cyano, Niederalkyl, Niederalkoxy oder Niederalkoxycarbonyl substituiertes Phenyl oder Benzyl,
$R_2$ und $R_3$ unabhängig voneinander Wasserstoff, Niederalkyl, Phenyl, Niederalkylphenyl, Niederalkoxyphenyl oder Benzyl, und $R_2$ auch Niederalkylcarbonyl, Niederalkylsulfonyl, Benzoyl oder Phenylsulfonyl,
$X_1$ und $X_2$ unabhängig voneinander Niederalkyl, Phenyl, Niederalkylphenyl, Niederalkoxyphenyl oder Benzyl und $X_1$ auch Wasserstoff, oder die Substituentenpaare
($R_2$ und $R_3$) und ($X_1$ und $X_2$), unabhängig voneinander, je zusammen mit dem sie verbindenden Stickstoffatom, Pyrrolidino, Piperidino oder Morpholino, und
$X_3$ Wasserstoff, Halogen, Niederalkyl oder Niederalkoxy bedeuten und die Ringe A, B und D unabhängig voneiander, unsubstituiert oder durch Cyano, Halogen, Niederalkyl oder Niederalkoxy substituiert sein können und der Ring D auch einen oder zwei ankondensierte Benzolringe enthalten kann.

5. Chinazolinverbindungen gemäß Anspruch 4, dadurch gekennzeichnet, daß in Formel (1) Y ein aminosubstituierter Phenylrest der Formel (1a) ist.

6. Chinazolinverbindungen gemäß einem der Ansprüche 4 und 5, dadurch gekennzeichnet, daß in Formel (1) Z Wasserstoff, $-OR_1'$, $-SR_1'$ oder $-NR_2R_3$ bedeutet.

7. Chinazolinverbindungen gemäß einem der Ansprüche 4 bis 6, dadurch gekennzeichnet, daß in Formel (1) Z die Gruppe $-OR_1'$ ist.

8. Chinazolinverbindungen gemäß Anspruch 4, dadurch gekennzeichnet, daß sie der Formel

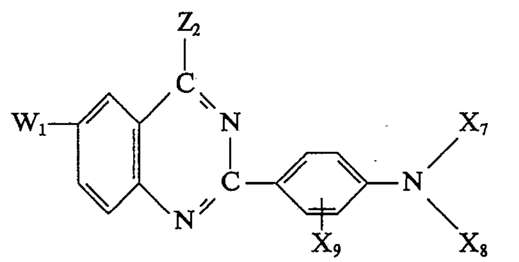

(3)

entsprechen, worin
$Z_2$ Wasserstoff, $R_7$, $-OR_7$, $-SR_7$ oder $-NR_8R_9$,
$R_7$ Niederalkyl, Niederalkoxy-niederalkyl, Cyclohexyl, Phenyl, Naphthyl, Benzyl oder durch Halogen, Cyano, Nitro, Methyl oder Methoxy substituiertes Phenyl,
$R_8$ und $R_9$ unabhängig voneinander Wasserstoff, Niederalkyl, Phenyl oder Benzyl und $R_8$ auch Niederalkylcarbonyl oder Benzoyl,
$X_7$ Nideralkyl, Phenyl, Niederalkylphenyl, Niederalkoxyphenyl oder Benzyl,
$X_8$ Wasserstoff, Niederalkyl, Phenyl oder Benzyl,
$X_9$ Wasserstoff, Methyl, Methoxy oder Äthoxy, und
$W_1$ Wasserstoff, Halogen, Methoxy oder Methyl
bedeuten.

9. Chinazolinverbindungen gemäß Anspruch 4, dadurch gekennzeichnet, daß sie der Formel

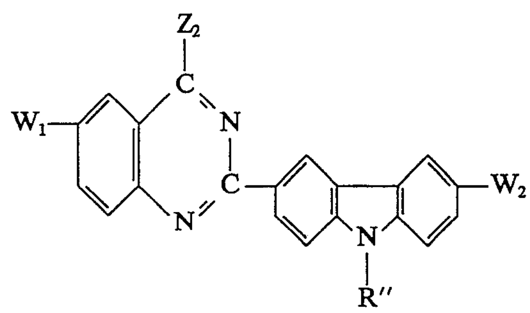

(4)

entsprechen, worin
$Z_2$ die in Anspruch 8 angegebene Bedeutung hat,
R'' Alkyl mit 1 bis 8 Kohlenstoffatomen oder Benzyl und

$W_1$ und $W_2$ unabhängig voneinander Halogen, Methoxy, Methyl oder besonders Wasserstoff bedeuten.

10. Verfahren zur Herstellung von Chinazolinverbindungen der im Anspruch 1 angegebenen Formel (1), in der Z $-OR_1'$, $-SR_1'$ oder $-NR_2R_3$ bedeutet, dadurch gekennzeichnet, daß man eine 4-Halogenchinazolinverbindung der Formel

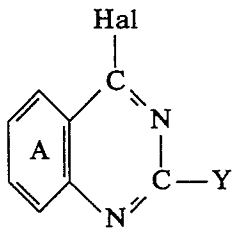

(5)

worin A und Y die im Anspruch 1 angegebene Bedeutung haben und Hal Halogen bedeutet mit einer Verbindung der Formeln

$R_1'-OH$ (6a) $R_1'-SH$ (6b) oder 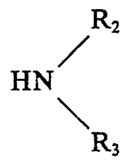 (6c)

umsetzt, worin $R_1'$, $R_2$ und $R_3$ die im Anspruch 1 angegebene Bedeutung haben.

11. Verfahren zur Herstellung von Chinazolinverbindungen der im Anspruch 1 angegebenen Formel (1), in der Z Wasserstoff bedeutet, dadurch gekennzeichnet, daß man eine 4-Halogenchinazolinverbindung der Formel

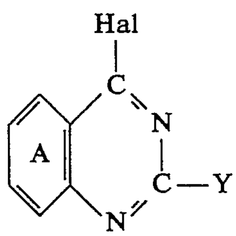

(5)

worin A und Y die im Anspruch 1 angegebene Bedeutung haben und Hal Halogen bedeutet, alkalisch enthalogeniert.

12. Verfahren zur Herstellung von Chinazolinverbindungen der in Anspruch 1 angegebenen Formel (1), in der Z den Rest $R_1$ bedeutet, dadurch gekennzeichnet, daß man eine Ketoamidoverbindung der Formel

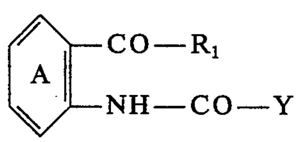

(11)

worin A, $R_1$ und Y die im Anspruch 1 angegebene Bedeutung haben, mit einer alkoholischen Ammoniaklösung reagieren läßt.

13. Verwendung einer Chinazolinverbindung der in einem der Ansprüche 1 bis 9 angegebenen Formel als Farbbildner in einem druckempfindlichen oder wärmeempfindlichen Aufzeichnungsmaterial.

14. Druck- oder wärmeempfindliches Aufzeichnungsmaterial, dadurch gekennzeichnet, daß es in seinem Farbreaktantensystem als Farbbildner mindestens eine Chinazolinverbindung der in einem der Ansprüche 1 bis 9 angegebenen Formel enthält.

**Claims**

1. A chromogenic quinazoline of the formula

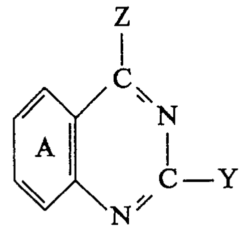

(1)

wherein Y is an amino-substituted phenyl radical of the formula

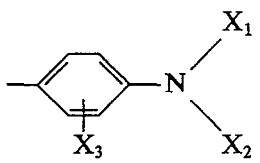

(1a)

or a 3-carbazolyl radical of the formula

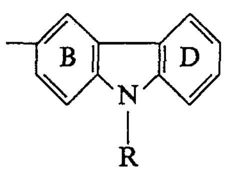

(1b)

and Z is hydrogen, $R_1$, $-OR_1'$, $-SR_1'$ or $-NR_2R_3$,
wherein R is hydrogen, alkyl of at most 12 carbon atoms which is unsubstituted or substituted by halogen, hydroxyl, cyano or lower alkoxy, or is alkenyl of at most 12 carbon atoms, acyl of 1 to 12 carbon atoms, benzyl or benzyl which is substituted by halogen, nitro, lower alkyl or lower alkoxy;
each of $R_1$ and $R_1'$ is alkyl of at most 12 carbon atoms which is unsubstituted or substituted by cyano or lower alkoxy, or is cycloalkyl, unsubstituted or substituted aryl or aralkyl or an unsubstituted or a substituted heterocyclic radical, and $R_1'$ is also haloalkyl of 2 to 6 carbon atoms;
each of $R_2$, $R_3$, $X_1$ and $X_2$ independently is hydrogen, alkyl of at most 12 carbon atoms which is unsubstituted or substituted by halogen, hydroxyl, cyano or lower alkoxy, or is cycloalkyl, phenyl, benzyl, or phenyl or benzyl each of which is substituted by halogen, nitro, cyano, lower alkyl, lower alkoxy or lower alkoxycarbonyl, and $R_2$ is also acyl of 1 to 12 carbon atoms, or each pair of substituents ($R_2$ and $R_3$) and ($X_1$ and $X_2$), together with the nitrogen atom to which said pair is attached, independently is a 5- or 6-membered heterocyclic radical;
$X_3$ is hydrogen, halogen, nitro, lower alkyl or lower alkoxy; and each of the rings A, B and D independently may be unsubstituted or substituted by cyano, nitro, halogen, lower alkyl, lower alkoxy or lower alkoxycarbonyl, and the ring D may also contain an unsubstituted or a substituted phenyl radical or a fused benzene ring, the term »lower« denoting those groups which contain 1 to 5 carbon atoms.

2. A quinazoline according to claim 1, wherein each of $R_1$ and $R_1'$ in formula (1) is alkyl of at most 12 carbon atoms which is unsubstituted or substituted by cyano or lower alkoxy, or is cycloalkyl, unsubstituted or substituted aryl or aralkyl, and $R_1'$ is also haloalkyl of 2 to 6 carbon atoms.

3. A quinazoline according to claim 1, wherein each of $R_1$ and $R_1'$ in formula (1) is a 5- or 6-membered heterocyclic radical of aromatic character which is unsubstituted or substituted by halogen, cyano, nitro, lower alkyl, lower alkoxy or lower alkoxycarbonyl.

4. A quinazoline according to claim 1, wherein R is alkyl of at most 12 carbon atoms which is unsubstituted or substituted by halogen, cyano or lower alkoxy, or is lower alkylcarbonyl, or benzyl which is unsubstituted or substituted by halogen, lower alkyl or lower alkoxy;
$R_1$ and $R_1'$ are of at most 8 carbon atoms which is unsubstituted or substituted by lower alkoxy, or is cyclohexyl, phenyl, naphthyl, benzyl, or phenyl or benzyl each of which is substituted by halogen, nitro, cyano, lower alkyl, lower alkoxy or lower alkoxycarbonyl;
each of $R_2$ and $R_3$ independently is hydrogen, lower alkyl, phenyl, lower alkylphenyl, lower alkoxyphenyl or benzyl, and $R_2$ is also lower alkylcarbonyl, lower alkylsulfonyl, benzoyl or phenylsulfonyl;
each of $X_1$ and $X_2$ independently is lower alkyl, phenyl, lower alkylphenyl, lower alkoxyphenyl or benzyl, and $X_1$ is also hydrogen; or

each pair of substituents ($R_2$ and $R_3$) and ($X_1$ and $X_2$), together with the nitrogen atom to which said pair is attached, independently is pyrrolidino, piperidino or morpholino;
$X_3$ is hydrogen, halogen, lower alkyl or lower alkoxy; and each of the rings A, B and D independently is unsubstituted or substituted by cyano, halogen, lower alkyl or lower alkoxy, and the ring D can also contain one or two fused benzene rings.

5. A quinazoline according to claim 4, wherein Y in formula (1) is an amino-substituted phenyl radical of the formula (1a).

6. A quinazoline according to either of claims 4 or 5, wherein Z in formula (1) is hydrogen, $-OR_1'$, $-SR_1'$ or $-NR_2R_3$.

7. A quinazoline according to any, one of claims 4 to 6, wherein Z in formula (1) is the $-OR_1'$ group.

8. A quinazoline according to claim 4 of the formula

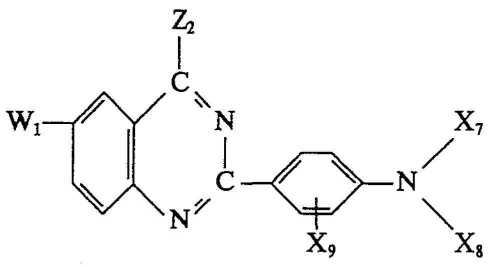

(3)

wherein $Z_2$ is hydrogen, $R_7$, $-OR_7$, $-SR_7$ or $-NR_8R_9$, and $R_7$ is lower alkyl, lower alkoxy-lower alkyl, cyclohexyl, phenyl, naphthyl, benzyl, or phenyl which is substituted by halogen, cyano, nitro, methyl or methoxy;
each of $R_8$ and $R_9$ independently is hydrogen, lower alkyl, phenyl or benzyl, and $R_8$ is also lower alkylcarbonyl or benzoyl; $X_7$ is lower alkyl, phenyl, lower alkylphenyl, lower alkoxyphenyl or benzyl; $X_8$ is hydrogen, lower alkyl, phenyl or benzyl; $X_9$ is hydrogen, methyl, methoxy or ethoxy; and $W_1$ is hydrogen, halogen, methoxy or methyl.

9. A quinazoline according to claim 4 of the formula

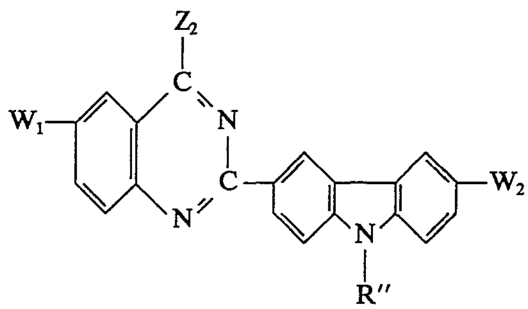

(4)

wherein $Z_2$ is as defined in claim 8, R″ is alkyl of 1 to 8 carbon atoms or benzyl, and each of $W_1$ and $W_2$ independently is hydrogen, halogen, methyl or methoxy.

10. A process for the production of a quinazoline of the formula (1) as indicated in claim 1, wherein Z is $-OR_1'$, $-SR_1'$ or $-NR_2R_3$, which process comprises reacting a 4-haloquinazoline of the formula

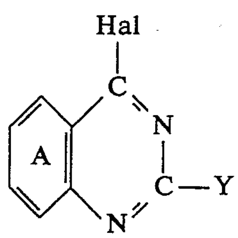

(5)

wherein A and Y are as defined in claim 1 and Hal is halogen, with a compound of the formula

$R_1'-OH$ (6a)

$R_1'-SH$ (6b)

or

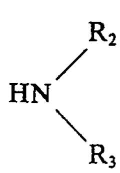

(6c)

wherein $R_1'$, $R_2$ and $R_3$ are as defined in claim 1.

11. A process for the production of a quinazoline of the formula (1) as indicated in claim 1, wherein Z is hydrogen, which process comprises dehalogenating a 4-haloquinazoline of the formula

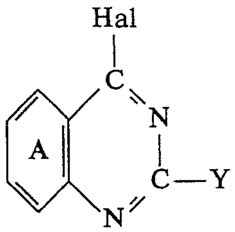

(5)

wherein A and Y are as defined in claim 1 and Hal is halogen, under alkaline conditions.

12. A process for the production of a quinazoline of the formula (1) as indicated in claim 1, wherein Z is the radical $R_1$, which process comprises reacting a ketoamido compound of the formula

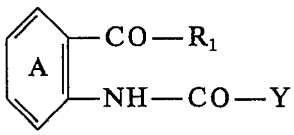

(11)

wherein A, $R_1$ and Y are as defined in claim 1, with a solution of Ammonia in alcohol.

13. Use of a quinazoline of the formula as indicated in any one of claims 1 to 9 as colour former in a pressure-sensitive or heat-sensitive recording material.

14. A pressure-sensitive or heat-sensitive recording material which contains in its colour reactive system, as colour former, at least one quinazoline of the formula as indicated in any one of claims 1 to 9.

**Revendications**

1. Dérivés chromogènes de la quinazoline de formule:

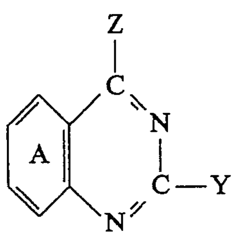

(1)

dans laquelle
Y désigne un radical phényle aminosubstitué de formule

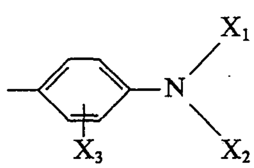

(1a)

ou un radical 3-carbazolyle de formule

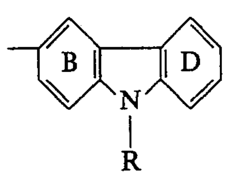

(1b)

et
Z désigne de l'hydrogène, $R_1$, $-OR_1'$ $-SR_1'$ ou $-NR_2R_3$, dans lesquels
R peut être de l'hydrogène, un alkyle ayant au maximum 12 atomes de carbone, non substitué ou substitué par un halogène, hydroxy, cyano ou alcoxy inférieur, alcényle ayant au maximum 12 atomes

de carbone, acyle ayant 1 à 12 atomes de carbone, benzyle ou benzyle substitué par un halogène, nitro, alkyle inférieur, alcoxy inférieur,

$R_1$ et $R_1'$ peuvent être chacun un alkyle ayant au maximum 12 atomes de carbone non substitué ou substitué par un cyano ou un alcoxy inférieur, un cycloalkyle, aryle ou aralkyle non substitué ou substitué ou radical hétérocyclique non substitué ou substitué, tandis que $R_1'$ peut également être un halogénoalkyle ayant 2 à 6 atomes de carbone,

$R_2$, $R_3$, $X_1$ et $X_2$ peuvent être indépendamment les uns des autres, un hydrogène, un alkyle ayant au maximum 12 atomes de carbone non substitué ou substitué par un halogène hydroxy cyano ou alcoxy inférieur, un cycloalkyle, phényle, benzyle ou encore un phényle ou benzyle substitué par un halogène, nitro, cyano, alkyle inférieur, alcoxy inférieur ou alcoxycarbonyle inférieur, et $R_2$ peut être également un acyle ayant 1 à 12 atomes de carbone, ou les paires de substituants ($R_2$ et $R_3$) et ($X_1$ et $X_2$) peuvent être chacune, indépendamment l'une de l'autre conjointement avec l'atome d'azote qui les relie, un radical hétérocyclique à 5 ou 6 chaînons,

$X_3$ désigne un hydrogène, halogène, nitro, alkylo inférieur ou alcoxy inférieur, tandis que les noyaux A, B et D, peuvent être indépendamment les uns des autres, non substitués ou substitués par un cyano, nitro, halogène, alkyle inférieur, alcoxy inférieur ou alcoxy carbonyle inférieur, tandis que le noyau D peut également présenter un radical phényle non substitué ou substitué ou un noyau benzène accolé par condensation, le terme »inférieur« se référant aux groupes ayant de 1 à 5 atomes de carbone.

2. Dérivés de quinazoline selon la revendication 1, caractérisés, par le fait que dans la formule (1) $R_1$ et $R_1'$ désigne chacun un alkyle ayant au maximum 12 atomes de carbone non substitués ou substitués par un cyano, ou un alcoxy inférieur, un cycloalkyle, aryle ou aralkyle non substitué ou substitué et $R_1'$ peut également un halogénoalkyle ayant 2 à 6 atomes de carbone.

3. Dérivés de quinazoline selon la revendication 1, caractérisés par le fait que dans la formule (1) $R_1$ et $R_1'$ désigne chacun un radical hétérocyclique à 5 ou 6 chaînons de nature aromatique, qui est non substitués ou substitués par un halogène, cyano, nitro, alkyle inférieur, alkoxy inférieur, ou alkoxycarbonyle inférieur.

4. Dérivés de quinazoline selon la revendication 1, caractérisés par le fait que R désigne un alkyle ayant au moins 12 atomes de carbone non substitués ou substitués par un halogène, cyano ou alcoxy inférieur, alkyle et carbonyle inférieur, ou benzyle non substitué ou substitué par un halogène alkyle inférieur ou alcoxy inférieur,

R et R' désignent chacun un alkyle ayant au maximum 8 atomes de carbone non substitué ou substitué par un alcoxy inférieur, un cyclohexyl, phényle, naphtyle, benzyle, ou un phényle ou benzyle substitué par un halogène, nitro, cyano, alkyle inférieur, alcoxy inférieur ou alcoxy carbonyle inférieur,

$R_2$ et $R_3$ désignent indépendamment l'un de l'autre, un hydrogène, alkyle inférieur, phényle, alkylphényle inférieur alcoxyphényle inférieur ou benzyle, et $R_2$ peut également désigner un alkyle carbonyle inférieur, alkylsulfonyl inférieur, benzoyle ou phénylsulfonyle,

$X_1$ et $X_2$ désignent indépendamment l'un de l'autre un alkyle inférieur, phényle, alkylphényle inférieur, alcoxyphényle inférieur ou benzyle, et $X_1$ peut désigner également un hydrogène, ou les paires de substituants

($R_2$ et $R_3$) et ($X_1$ et $X_2$), désignant indépendamment l'une de l'autre, chacune conjointement avec l'atome d'azote qui les relie, un cycle pyrrolidino, pipéridino ou morpholino, et

$X_3$ désigne un hydrogène, halogène, alkyle inférieur ou alcoxy inférieur, et les noyau A, B et D peuvent être, indépendamment les uns des autres, non substitués ou substitués par un cyano, halogène, alkyle inférieur ou alcoxy inférieur, tandis que le noyau D peut renfermer un ou deux noyaux benzène accolés par condensation.

5. Dérivés de quinazoline selon la revendication 4, caractérisés par le fait que dans la formule (1) Y est un radical phényle aminosubstitué de formule (1a).

6. Dérivés de quinazoline selon la revendication 4 ou 5, caractérisés par le fait que dans la formule (1) Z désigne un hydrogène, $-OR_1'$, $-SR_1'$ ou $-NR_2R_3$.

7. Dérivés de quinazoline selon l'une quelconque des revendications 4 à 6, caractérisés par le fait que dans la formule (1), Z est le groupe $-OR_1'$.

8. Dérivés de quinazoline selon la revendication 4, caractérisés qu'ils correspondent à la formule

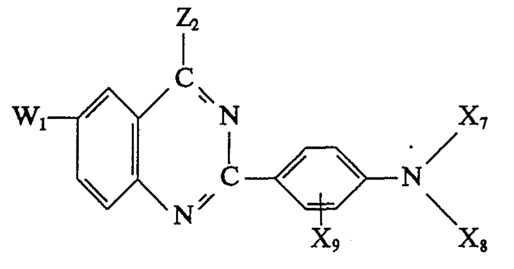

(3)

dans laquelle $Z_2$ désigne un hydrogène, $R_7$, $-OR_7$, $-SR_7$ ou $-NR_8R_9$,

$R_7$ désigne un alkyle inférieur, alcoxy inférieur-alkyle inférieur, cyclohexyle, phényle, naphthyle,

benzyle ou phényle substitué par un halogène, cyano, nitro, méthyle ou méthoxy,
$R_8$ et $R_9$ désignent indépendamment l'un de l'autre un hydrogène, alkyle inférieur, phényle ou benzyle et $R_8$ désigne également un alkylcarbonyle inférieur ou benzoyle,
$X_7$ désigne un alkyle inférieur, phényle, alkylphényle inférieur, alcoxyphényle inférieur ou benzyle,
$X_8$ désigne un hydrogène, alkyle inférieur, phényle ou benzyle,
$X_9$ désigne un hydrogène, méthyle, méthoxy ou éthoxy, et
$W_1$ désigne un hydrogène, halogène, méthoxy ou méthyle.

9. Dérivés de quinazoline selon la revendication 4, caractérisés par le fait qu'ils correspondent à la formule

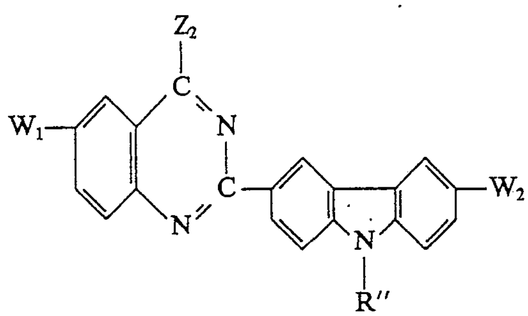

(4)

dans laquelle
$Z_2$ est défini comme spécifié dans la revendication 8,
R″ désigne un alkyle ayant 1 à 8 atomes de carbone ou un benzyle et
$W_1$ et $W_2$ désigne indépendamment l'un de l'autre un halogène, méthoxy, méthyle ou en particulier hydrogène.

10. Procédé pour la préparation des dérivés de quinazoline de formule (1) indiquée dans la revendication 1, dans laquelle Z désigne $-OR_1'$, $-SR_1'$ ou $-NR_2R_3$, caractérisé par le fait que l'on fait réagir un dérivé de 4-halogenoquinozaline de formule

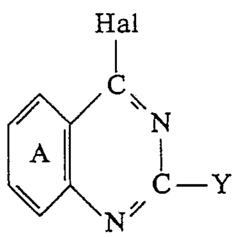

(5)

dans laquelle A et Y sont définis comme spécifié dans la revendication 1 et Hal désigne un halogène, avec un composé des formules

$R_1'-OH$ (6a)   $R_1'-SH$ (6b)   ou   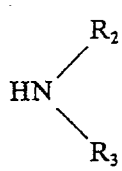 (6c)

dans lesquelles, $R_1'$, $R_2$ et $R_3$ sont définis comme spécifié dans la revendication 1.

11. Procédé de préparation de dérivés de quinazoline de formule (1) indiquée dans la revendication 1, dans laquelle Z désigne un hydrogène, caractérisé par le fait que l'on soumet à une déshalogénation alcaline, un dérivé 4-halogénoquinazoline de formule

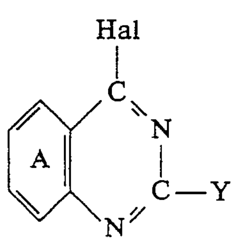

(5)

dans laquelle A et Y sont définis comme spécifié dans la revendication 1 et Hal désigne un halogène.

12. Procédé de préparation de dérivés de quinazoline de formule (1) indiquée à la revendication 1, dans laquelle Z désigne le radical $R_1$, caractérisé par le fait que l'on laisse réagir un composé céto-amido de formule

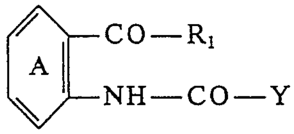

(11)

dans laquelle A, $R_1$ sont définis comme spécifié dans la revendication 1, avec une solution d'ammoniac alcoolique.

13. Utilisation d'un dérivé de quinazoline selon les formules indiquées dans l'une quelconque des revendications 1 à 9, comme l'agent colorant, par développement de couleur, dans un matériau d'enregistrement sensible à la pression ou sensible à la chaleur.

14. Matériau d'enregistrement sensible à la pression ou à la chaleur, caractérisé par le fait qu'il contient comme un formateur de couleur ou agent chromogène dans son système de réactifs colorants, au moins un dérivé de quinazoline de formule indiquée dans l'une quelconque des revendications 1 à 9.